Europäisches Patentamt

European Patent Office

Office européen des brevets

(19)

(11) Veröffentlichungsnummer: **0 268 858 B1**

# (12) EUROPÄISCHE PATENTSCHRIFT

(45) Veröffentlichungstag der Patentschrift: **09.10.91**

(51) Int. Cl.5: **A61F 13/15**

(21) Anmeldenummer: **87115777.2**

(22) Anmeldetag: **27.10.87**

(54) Verfahren zum Herstellen von auf dem Körper zu tragenden Artikeln.

(30) Priorität: **27.10.86 JP 254892/86**
**28.01.87 JP 19338/87**

(43) Veröffentlichungstag der Anmeldung:
**01.06.88 Patentblatt 88/22**

(45) Bekanntmachung des Hinweises auf die
Patenterteilung:
**09.10.91 Patentblatt 91/41**

(84) Benannte Vertragsstaaten:
**DE ES FR IT NL SE**

(56) Entgegenhaltungen:
**EP-A- 0 109 126**
**EP-A- 0 190 881**
**EP-A- 0 219 326**
**EP-A- 0 243 013**
**GB-A- 2 161 059**

(73) Patentinhaber: **UNI-CHARM CORPORATION**
**182, Shimobun Kinsei-cho**
**Kawanoe-shi Ehime-ken(JP)**

(72) Erfinder: **Igaue, Takamitsu**
**385-1-3, Handa-otsu Kaneda-cho**
**Kawanoe-shi Ehime-ken(JP)**
Erfinder: **Tanji, Hiroyuki**
**2529-229, Kawanoe-cho**
**Kawanoe-shi Ehime-ken(JP)**

(74) Vertreter: **Sperling, Rüdiger, Dipl.-Ing. et al**
**Patentanwälte Dipl.Ing.S. Staeger**
**Dipl.Ing.Dipl.Wirtsch.Ing. R Sperling Müller-**
**strasse 31**
**W-8000 München 5(DE)**

## Beschreibung

Die Erfindung betrifft ein Verfahren zum Herstellen von auf dem Körper zu tragenden Artikeln, wie beispielsweise Wegwerf-Höschenwindeln, Windelhosen und ähnliches, insbesondere ein Verfahren zum Ausbilden von Seitenklappen einander gegenüberliegenden Seiten eines solchen Artikel und zum Einarbeiten von elastischen Teilen in diese Seitenklappen, die einem genauem Paßsitz um die Schenkel des Trägers dienen.

Aus der älteren Anmeldung EP 87 302 764 ist es bekannt, an Wegwerfwindeln, Seitenklappen mit hoher Flexibilität an einander gegenüberliegenden Seiten der Windel auszubilden und in diese Seitenklappen elastische Teile einzuarbeiten, um damit einen genauen Paßsitz um die Schenkel des Trägers, beispielsweise eines Babies sicherzustellen, so daß diese Wegwerfwindel generell beim Gebrauch die Form eines Höschens aufweist; derartige Höschenwindeln sind die zur Zeit am meisten verwendeten Windeln.

Die Seitenklappen einer solchen Windel weisen allgemein Abschnitte einer oberen Lage und einer rückseitigen Lage auf, die sich von einander gegenüberliegenden Seiten eines Saugkörpers weg erstrecken dessen obere und untere Seite jeweils mit der oberen und der rückseitigen Lage der Windel bedeckt ist. Die jeweiligen Lagen, die auf diese Weise die Seitenklappen bilden, sind jeweils kontinuierlich. Die Herstellung der Seitenklappen aus den oberen und unteren Lagen des Artikels, z.B. eine Wegwerfwindel, der aus ökonomischen Gründen ein Massenprodukt mit hoher Ausstoßrate ist, wirft verschiedene Probleme auf, die nachfolgend näher erläutert werden:

a) Es ist möglich, Seitenklappen aus einem zu dem der oberen und/oder der rückseitigen Lage unterschiedlichem Material herzustellen, z.B. einem Material, das in seiner Luftdurchlässigkeit günstiger ist als der der rückseitigen Lage, um die Schutzwirkung vor Dampfbildung im Inneren des Artikels zu verbessern.

b) Wenn es gewünscht wird, die Seitenklappen mit einem besonderem Aufbau auszustatten, um sicherzustellen, daß die Seitenklappen in engem Kontakt an den Schenkeln des Babies anliegen, um dabei jeglichen Ausfluß von Ausscheidungen zu verhindern, so ist es in der Praxis schwierig, einen derartigen Aufbau auf der Produktionslinie gleichzeitig mit dem Ausbilden des Hauptkörpers der einzelnen Artikel zu realisieren. Selbst wenn es möglich sein sollte, dies durchzuführen, so ist die Art des Betriebsablaufs nicht geeignet für Wegwerfartikel, die aus wirtschaftlichen Gründen eine Hochgeschwindigkeitsmassenproduktion erfordern.

c) Nachteile, ähnlich denjenigen, die in b) aufgeführt worden sind, treten auch dann auf, wenn es gewünscht wird, verschiedene Faktoren, wie beispielsweise die Breite der Seitenklappen und den Abstand zwischen den einander gegenüberliegenden elastischen Teilen wahlweise im vorderen Bereich und im rückwärtigen Bereich des Artikels zu variieren.

Aus der GB 2 161 059 ist eine Windelhose bekannt, bei der aus dem Material der wasserdurchlässigen, innenliegenden Lage einstückig Klappen ausgeformt sind, die sich im angelegten Zustand aufrichten. Die auf diese Weise ausgebildete Seitenklappe ist deshalb nachteilig weil sie einen erhöhten Herstellungsaufwand erfordert und zum anderen keinen wasserdichten Abschluß gewährleistet, da die Klappe aus dem Material der wasserdurchlässigen inneren, dem Körper zugewandten Lage besteht.

Der Erfindung liegt daher die Aufgabe zugrunde, ein Verfahren zum Herstellen von Artikeln der genannten Gattung so zu verbessern, daß es ermöglicht wird, die oben genannten Probleme wirkungsvoll zu lösen.

Die Aufgabe wird erfindungsgemäß mit einem Verfahren zum Herstellen von auf dem Körper zu tragenden Artikeln, wie beispielsweise Windelhöschen durch die folgenden Verfahrenschritte gelöst:

a) Ausbilden eines Hauptkörpers mit sich auf einander gegenüberliegenden Seiten nach außen erstreckenden ersten Seitenklappen,

b) Ausbilden von zweiten Seitenklappen, die vom Hauptkörper getrennt sind,

c) Anbringen von elastischen Teilen an den zweiten Seitenklappen entlang jeweils eines Seitenrands,

d) Verbinden jeder zweiten Seitenklappe mit der Oberfläche der zugeordneten Seitenklappe entlang des anderen Seitenrands und deren in Längsrichtung einander gegenüberliegenden Enden derart, daß der mit dem elastischen Teil versehene Seitenrand der zweiten Seitenklappen nach außen weist.

Entsprechend der vorliegenden Erfindung werden einerseits der Hauptkörper des Artikels und andererseits die zweiten Seitenklappen, die am Hauptkörper auf einander gegenüberliegenden Seiten als komplementäre Abschnitte der vollständigen Seitenklappen anzuordnen sind und die mit den zugeordneten elastischen Teilen ausgestattet sind, separat voneinander hergestellt und anschließend die letzteren auf den ersteren an den einander gegenüberliegenden Seiten aufgelegt. Auf diese Weise kann das eingangs beschriebene Problem wirksam gelöst werden. Wenn die gesamten Seitenklappen von den Abschnitten der oberen und/oder rückseitigen Lage, d.h. den Komponenten des Hauptkörpers gebildet werden, die sich voneinander gegenüberliegenden Seiten des Hauptkörpers wegerstrecken, so haben die Seitenklappen die gleichen luftdurchlässigen Eigenschaften wie die Komponenten des Hauptkörpers. Entsprechend der vorliegenden Erfindung werden die zweiten Seitenklappen aus einem Material hergestellt, dessen Luftdurchlässigkeit besser ist als die der Komponenten der Hauptkörper, wodurch die Wirksamkeit beim Verhindern einer Dampfbildung oder des Ansammelns von stickiger Luft in der Windel verbessert wird.

Die zweiten Seitenklappen können entlang der Faltabschnitte mit elastischen Teilen versehen sein; in Längsrichtung einander gegenüberliegende Enden der gefalteten Abschnitte können fixiert sein, um wahlweise diese gefalteten Abschnitte aufzurichten, niederzulegen oder zu neigen. Die vorliegende Erfindung ermöglicht es, derartige Seitenklappen in relativ einfacher Weise herzustellen.

Des weiteren ist ein Abstand der in dem Artikel eingearbeiteten elastischen Teilen, vorzugsweise zumindest im rückwärtigen Bereich breiter oder weiter als im Zentral-d.h. Schrittbereich, um den Paßsitz des Artikel an den Schenkeln eines Trägers sicherzustellen. Dies wird durch einen genauen Breitenbereich und Winkel sichergestellt, mit dem die Seitenklappen gefaltet werden, wie auch durch die Breite und den Winkel über und mit welchem die nicht gefalteten Abschnitte der zweiten Seitenklappen an den jeweiligen ersten Seitenklappen des Hauptkörpers befestigt werden.

Die vorliegende Erfindung ermöglicht also ebenfalls, derartige Seitenklappen auf relativ einfache Weise herzustellen.

Durch die erfindungsgemäße Ausbildung ist es möglich, Seitenklappen des relativ speziellen erwähnten Aufbaus in den Artikel einzubauen, ohne eine signifikante Reduzierung der gewünschten Produktivitätsrate.

Im folgenden wird die Erfindung anhand in den Zeichnungen dargestellter Ausführungsbeispiele näher erläutert. Es zeigen:

Fig.1-5: Eine Folge von Draufsichten auf ein erstes Ausführungsbeispiel einer erfindungsgemäßen Wegwerfwindel, wobei der Herstellungsprozeß sequentiell dargelegt ist;

Fig. 6 eine Draufsicht auf eine vollständig ausgerüstete Windel,

Fig. 7 eine perspektivische Ansicht der Windel aus Fig. 6,

Fig. 8 eine Querschnitt durch die Windel aus Fig. 6,

Fig. 9 eine perspektivische Ansicht eines Teils der Winddel, die einen Abschnitt der Windel aus Fig. 6 mit einer Seitenklappe darstellt,

Fig.10-14 eine Folge von Draufsichten auf ein zweites Ausführungsbeispiel einer erfindungsgemäßen Wegwerfwinel, wobei der Herstellungsprozeß sequentiell dargelegt ist.

Fig. 15 eine Draufsicht auf eine vollständig ausgerüstete Windel,

Fig. 16 eine perspektivische Ansicht der Windel aus Fig. 15,

Fig. 17 eine Ansicht auf einen Querschnitt durch die Windel aus Fig. 15, und

Fig. 18 eine perspektivische Ansicht eines Teils der Windel, die einen Abschnitt der Windel aus Fig. 6 mit einer Seitenklappe darstellt.

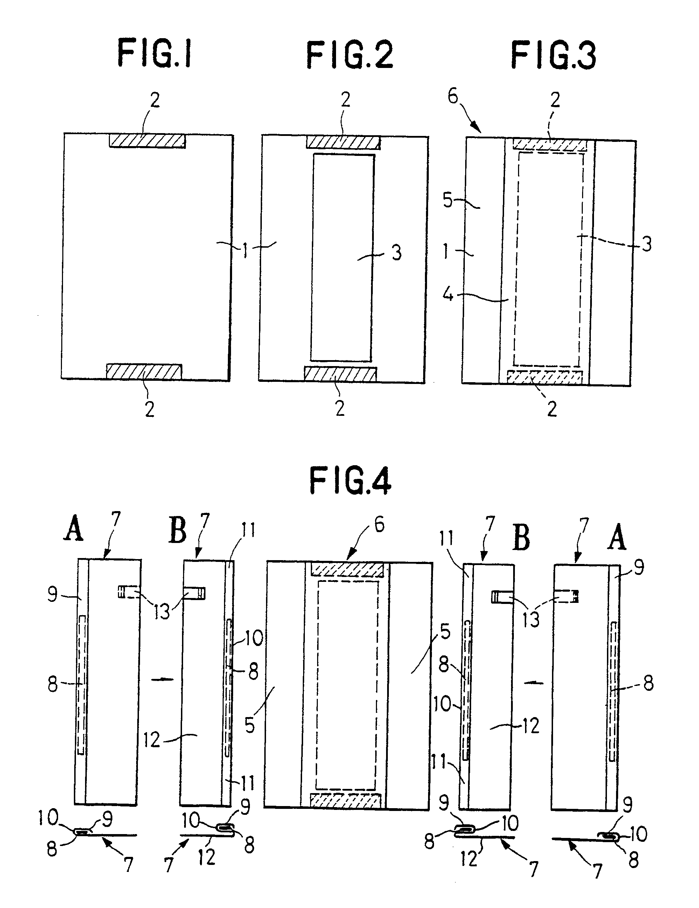

Die Figuren 1 bis 9 zeigen ein erstes Ausführungsbeispiel der vorliegenden Erfindung. Es wird zunächst Bezug genommen auf die Figuren 1 bis 3. An in Längsrichtung einander gegenüberliegenden Rändern einer wasserundurchlässigen rückseitigen Lage 1 sind jeweils mittig auf der Oberfläche elastische Teile 2 angeordnet, die einem gewünschten Paßsitz um die Hüften des Trägers dienen. In einem Gebiet, das zwischen den genannten einander gegenüberliegenden elastischen Teilen 2 liegt, ist auf der Oberfläche der rückwärtigen Lage 1 ein Saugkörper 3 befestigt. Die Befestigung der elastischen Teile 2, wie auch des Saugkörpers 3 geschieht vorzugsweise durch ein Klebemittel.

Die elastischen Teile 2 und der Saugkörper 3 werden anschließend mit einer wasserdurchlässigen Oberlage 4 abgedeckt, die etwas größer ist, als der Saugkörper 3. Die Befestigung geschieht ebenfalls vorzugsweise durch ein Klebemittel. Auf diese Weise wird ein Hauptkörper 6 der Windel ausgebildet, an dem erste Seitenklappen 5 sich von den einander gegenüberliegenden Rändern des Saugkörpers 3 nach außen erstercken.

Es wird nun auf die Figur 4 Bezug genommen. Getrennt von dem Hauptkörper 6 werden zweite Seitenklappen 7 aus einer wasserundurchlässigen Lage, die wesentlich schmäler ist als die rückwärtige Lage 1 gebildet. Jede dieser Seitenklappen 7 ist entlang eines Seitenrands 9 mit einem eingeklebten Elastikteil 8 für einen Paßsitz um den Schenkel eines Trägers ausgestattet. Der Seitenrand 9 wird anschließend umgefaltet, um das elastische Teil 8 zu überdecken (Fig. 4A).

Nach einem Wenden der Seitenklappe 7 wird der das elastische Teil 8 enthaltende Randabschnitt nach außen gefaltet (Fig. 4B) und der gefaltete Abschnitt 10 entlang seiner in Längsrichtung einander gegenüberliegenden Enden durch Ankleben auf der Oberfläche des nicht gefalteten Abschnitts 12 befestigt. Die zweite Seitenklappe 7 ist des weiteren auf der dem Seitenrand mit dem elastischen Teil abgewandten Seite neben einem der sich in Längsrichtung gegenüberliegenden Enden mit einem Befestigungsband 13 versehen, das dem Anlegen der Windel um die Hüften dient. Das Anbringen des Befestigungsbands 13 kann vor oder nach dem Falten des Abschnitts 10 geschehen. Die Herstellung der zweiten Seitenklappe 7 ist nun beendet.

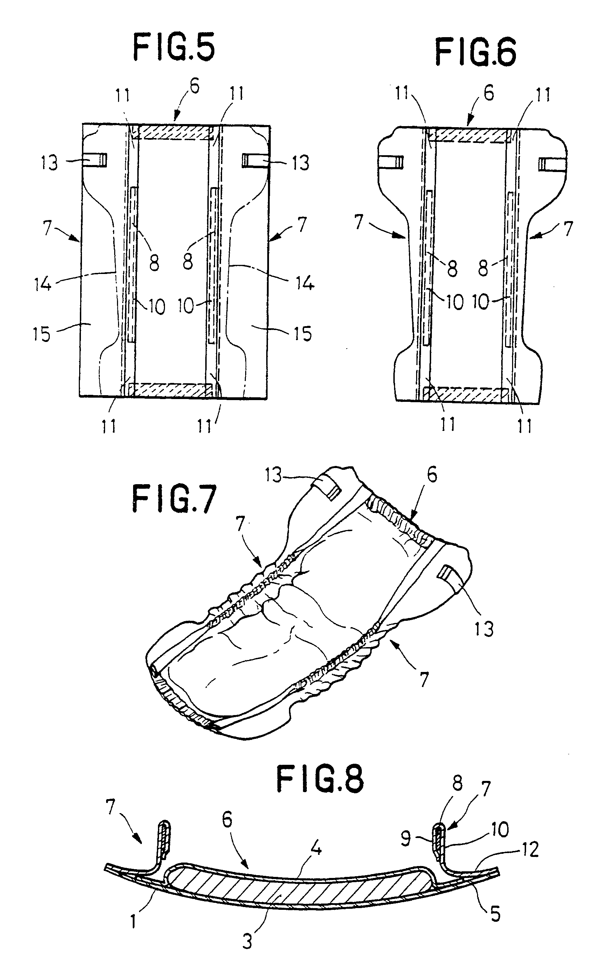

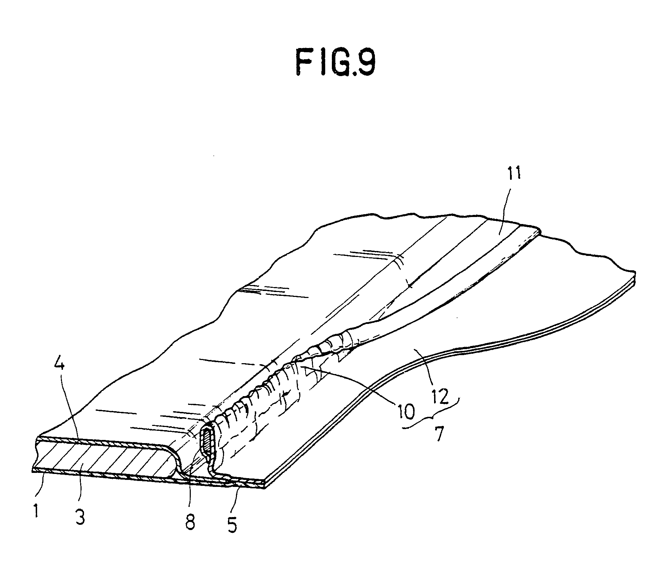

Es wird nun auf die Figuren 5 und 6 Bezug genommen. Die zweiten Seitenklappen 7 werden nun entlang der nicht gefalteten Bereiche 12 mit ihrer Rückseite nach unten weisend auf die entsprechende erste Seitenklappe 5 des Hauptkörpers gelegt und vorzugsweise mit einem Kleber verbunden, so daß die jeweiligen elastischen Teile 8 sich an den Innenseiten befinden (Fig. 7 und 9). Ein derartiges Verbinden findet entlang solchen Linien statt, wie sie mit der unterbrochenen Linie 14 dargestellt ist, oder in Bereichen 15, die sich jeweils außen jenseits der Linien erstrecken. Anschließend werden die Bereiche 15 entlang der jeweiligen Linien 14 weggeschnitten, so daß die ersten und zweiten Seitenklappen 5 und 7 eine Konfiguration aufweisen, die einen gewünschten Paßsitz am Körper des Trägers begünstigen.

Das Falten der Abschnitte 10 und das Befestigen des Befestigungsbands 13 an den sich in Längsrichtung einander gegenüberliegenden Enden kann auch während oder nach dem Befestigen der zweiten Seitenklappen 7 am Hauptkörper 6 durchgeführt werden.

Die Figurenn 10 bis 18 zeigen ein zweites Ausführungsbeispiel der vorliegenden Erfindung.

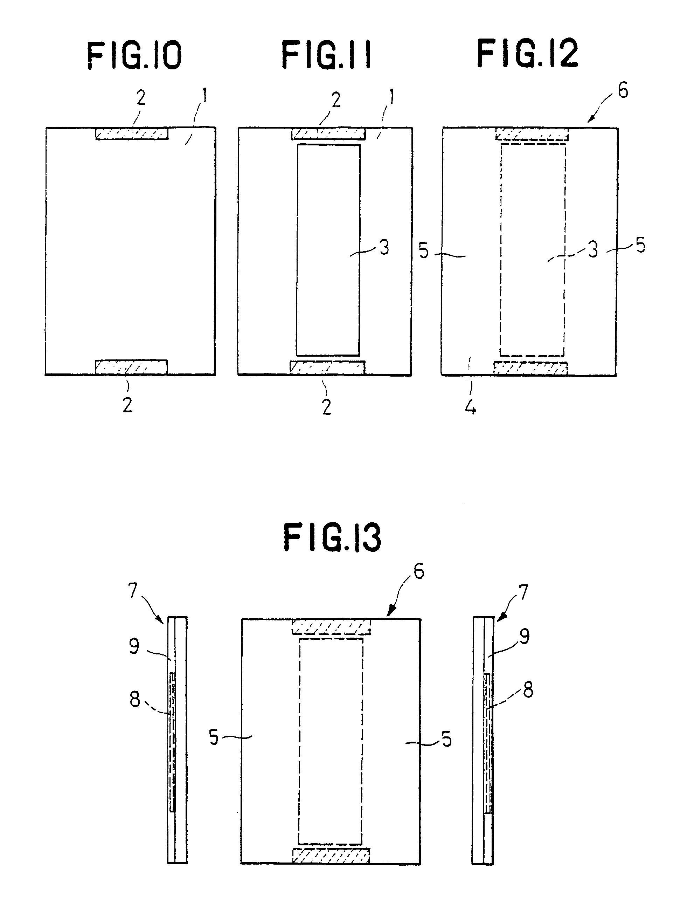

Es wird zunächst auf die Figuren 10 bis 12 Bezug genommen. Der Hauptkörper des zweiten Ausführungsbeispiels besteht im wesentlichen aus den gleichen Elementen wie der Hauptkörper aus dem ersten Ausführungsbeispiel, der Aufbau geschieht ähnlich wie im Zusammenhang mit den Figuren 1 bis 3 beschrieben. Der wesentliche Unterschied besteht darin, daß die wasserdurchlässige Oberlage 4 im vorliegenden Ausführungsbeispiel die gleiche Abmessung besitzt, wie die wasserundurchlässige rückseitige Lage 1.

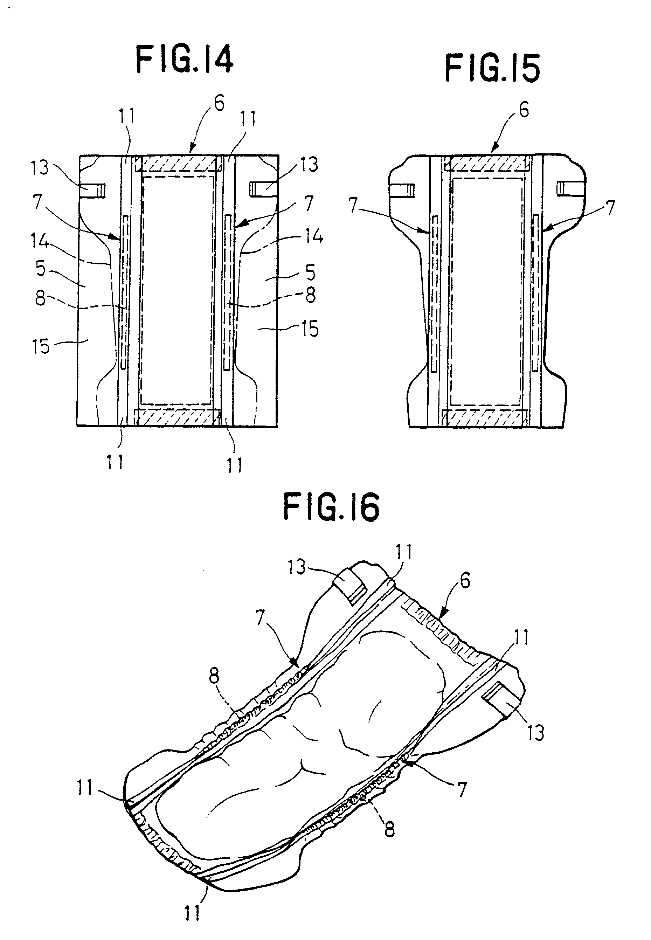

Wie aus Fig. 13 zu erkennen ist, werden die zweiten Seitenklappen 7 getrennt vom Hauptkörper 6 aus einer wasserundurchlässigen Lage hergestellt und weisen eine Breite auf, die wesentlich schmäler ist als die rückseitige Lage 1. Jede der zweiten Seitenklappen 7 ist entlang eines Seitenrands 9 mit einem elastischen Teil 8 versehen, das einem Paßsitz der Windel um den Schenkel eines Trägers dient. Das elastische Teil ist vorzugsweise aufgeklebt und anschließend dieser Seitenrand 9 umgefaltet, um das elastische Teil abzudecken. Nach dem Umfalten ist die Herstellung der zweiten Seitenklappen beendet.

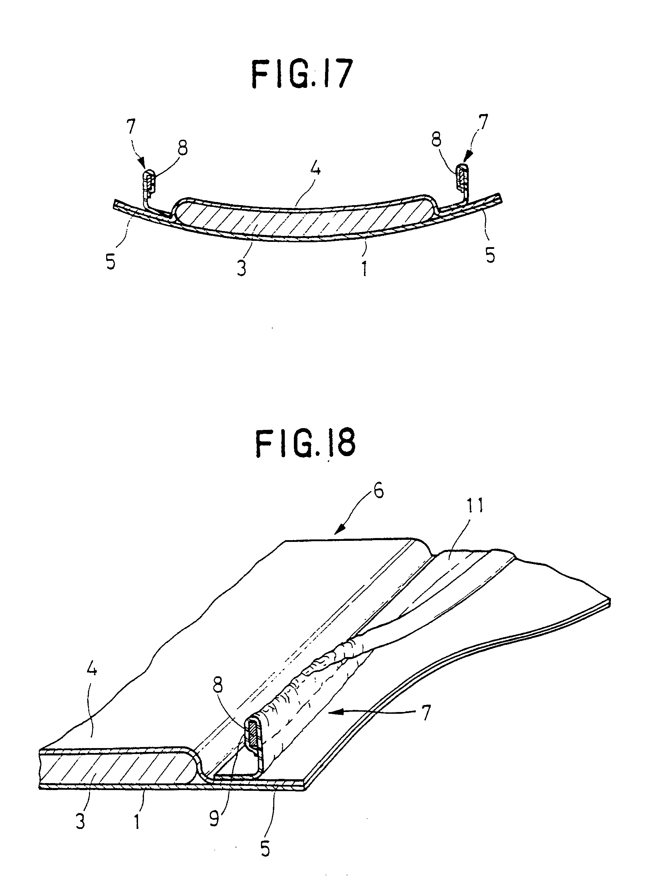

Es wird nun auf die Figuren 14 und 15 Bezug genommen. Jede der zweiten Seitenklappen 7 wird so orientiert, daß die Seitenkante 9, die das zugeordnete elastische Teil 8 enthält, nach außen gerichtet ist. Dann wird jede zweite Seitenklappe entlang der inneren Randkante und den in Längsrichtung einander gegenüberliegenden Enden des Seitenrands, der die zugeordneten elastischen Teile 8 aufweist, mit der Bodenfläche dieser Komponenten nach unten weisend, auf die Oberfläche der enstprechenden ersten Seitenklappe 5 des Hauptkörpers, vorzugsweise mit einem Klebemittel, aufgeklebt (Fig. 16 bis 18). Anschließend werden die äußeren Bereiche 15, die sich außen jenseits der Linien 14 erstrecken, entlang dieser Linien 14 jeweils weggeschnitten, so daß die ersten Seitenklappen 5 eine Konfiguration erhalten, die für einen gewünschten Paßsitz am Körper des Trägers günstig ist.

Jede der ersten Seitenklappen 5 ist in der Nähe eines ihrer in Längsrichtung einander gegenüberliegenden Enden mit einem Befestigungsband 13 derart versehen, daß dieses Befestigungsband 13 seitlich gegenüber dem entsprechenden Befestigungsband 13 angeordnet ist, das sich auf der anderen ersten Seitenklappe 5 befindet. Bei einer Anordnung, in der einander gegenüberliegenden Seitenränder der ersten Seitenklappen 5 mit den gegenüberliegenden Seitenrändern der zweiten Seitenklappe 7 zusammenfallen, wird das Befestigungsband 3 an beiden, den ersten Seitenklappen und den zweiten Seitenklappen angeordnet. Die rückseitige Lage 1 kann aus einem Material, wie beispielsweise ein luftdurchlässiger Kunststoff oder aus einem Laminat, aus diesem Kunststofffilm und einem Faservlies bestehen. Die elastischen Teile 2 und 8 können ein Polyurethanschaum oder ein Gummi sein, die obere Lage 4 ein Material, wie beispielsweise ein Faservlies oder ein poröser Kunststofffilm. Das Material der zweiten Seitenklappen kann ein luftdurchlässiger Kunststofffilm, ein Laminat aus einem solchen Film und einem Faservlies oder ein Faservlies sein, das mit einem geeigneten Repellent behandelt worden ist.

Wenn die zweiten Seitenklappen aus einem Material wie beispielsweise wasserdichtem Vlies oder einem Laminat aus einem Kunststofffilm und einem Faservlies bestehen, und Berührflächen aufweisen, die unterschiedlich sind, je nachdem ob es sich um ihre Ober- oder Unterseite handelt, so kann die Komponente, die ein angenehmes Berührungsgefühl aufweist, beispielsweise ein Faservlies so ausgebildet sein, daß sie in direktem Kontakt mit der Haut des Körpers kommt.

Obwohl die Ausführungsbeispiele so dargestellt worden sind, daß die zweiten Seitenklappen 7 auf einander gegenüberliegenden Seiten der Windel in einem gleichförmigen Abstand entlang des vorderen, des Schritt- und des rückwärtigen Bereichs der Windel mit dem Hauptkörper verbunden sind, so ist es jedoch auch beabsichtigt und liegt im Bereich der Erfindung, die beiden Seitenklappen 7 so mit dem Hauptkörper zu verbinden, daß sich dieser Abstand vom vorderen Bereich zum hinteren Bereich sich graduell vergrößert. Auf diese Weise ist der Abstand der elastischen Teile 8, die an den gegenüberliegenden Seiten der Windel angeordnet sind, auch von dem vorderen Bereich zum rückwärtigen Bereich vergrößert, was zu einem notwendigen gegenseitigen Abstand der elastischen Teile 8 führt, um einem genauen Paßsitz auf den Hüften des Träger sicherzustellen, wobei der Abstand dort weiter ist als im Schritt- und im Bauchbereich.

Obwohl es für die Erfindung nicht erforderlich ist, Windeln mit dem elastischen Teil 2 für die Hüfte auszustatten, so ist es doch günstig, diese elastischen Teile 2 entlang den in Längsrichtung einander gegenüberliegenden Rändern der rückseitigen Lage 1 und der Oberlage 4 zu verwenden. Hinsichtlich des üblichen Herstellprozesses der Windel, während dem die rückseitige Lage 1 und die Oberlage 4 kontinuierlich in Richtung der Produktionslinie angeliefert werden und die kontinuierliche Windelbahn nach dem Vervollständigen in individuelle Einzelwindeln geschnitten werden, werden die elastischen Teil 2 für die Hüfte, die jeweils eine Breite aufweisen, die dem doppelten der endgültigen Breite der individuellen elastischen Teile entspricht, so angeordnet, daß ihre in Längsrichtung des Gummis verlaufende Mittellinie gerade mit den Schnittlinien zusammenfällt. Auf diese Weise werden die jeweiligen elastischen Teile, die zunächst die doppelte Breite aufweisen, in zwei Teile gleicher Breite aufgeteilt, wenn die Windelbahn in die einzelnen Windeln geschnitten wird, wobei die eine Hälfte der doppelt breiten elastischen Teile der vorangehenden Windel, die zweite Hälfte der nachfolgenden Windel zugeteilt wird. Auf diese Weise wird das Anbringen der elastischen Teile wirkungsvoll verbessert.

Die einander gegenüberliegenden Seiten der Oberlage 4 können zwischen der Bodenfläche des Saugkörpers 3 und der rückseitigen Lage 1 entlang der gegenüberliegenden Seiten des Saugkörpers 3 eingeschlagen werden, wenn dies notwendig oder als geeignet angesehen wird.

In der auf diese Weise hergestellten Windel werden die zweiten Seitenklappen 7, außer ihre einander gegenüberliegenden Längsenden 11, aufgerichtet, wenn die jeweiligen elastischen Teile sich im zusammengezogenen Zustand befinden, so, wie in den Figuren 7 bis 9 und den Figuren 16 bis 18 dargestellt ist. Wenn die elastischen Teile 8 jedoch genügend gestreckt werden, wie es in den Figuren 6 und 15 gezeigt ist, beispielsweise als Ergebnis eines Längszugs an der Windel, so fallen die zweiten Seitenklappen 7 nach außen. Daher werden die zweiten Seitenklappen mit einem bestimmten, nach außen geneigten Winkel versehen, jedoch ohne vollständig zusammenzufallen und zwar insoweit als die elastischen Teile 8 in einem moderaten gedehnten Zustand gehalten werden.

Die spezielle Anordnung, nämlich daß die äußeren Seitenränder die zugeordneten elastischen Teile 8 enthalten, nach außen gerichtet und an den in Längsrichtung einander gegenüberliegenden Enden befestigt sind, ist der Grund dafür, daß die zweiten Seitenklappen 7 aufgerichtet, vollständig niedergelegt oder geneigt werden können, was jeweils vom Ausmaß der Dehnung der elastischen Teile 8 abhängt. Eine solche Funktion der zweiten Seitenklappen 7 ist sehr wirksam, um diese Seitenklappen eng um die Schenkel des Trägers zu legen und um eine zuverlässige Anlage um die Schenkel aufrechtzuerhalten, selbst wenn der Träger, beispielsweise ein Baby, mit den Beiden strampelt. Die erfindungsgemäße Ausbildung schützt dann in jedem Fall vor einem Austreten irgendwelcher Körperausscheidungen aus der Windel.

## Patentansprüche

1.  Verfahren zum Herstellen von Wegwerfunterhosen, insbesondere Windelhosen, Mit den folgenden Verfahrensschritten:

    a) Ausbilden eines Hauptkörpers (6) mit Sich auf einander gegenüberliegenden Seiten nach außen erstreckenden ersten Seitenklappen (5),

    b) Ausbilden von zweiten Seitenklappen (7), die vom Hauptkörper (6) getrennt sind,

    c) Anbringen von elastischen Teilen (8) an den zweiten Seitenklappen (7) englang jeweils eines Seitenrandes,

    d) Verbinden der Oberfläche der zugeordneten ersten Seiten-klappe (5) mit zum einen jeder zweiten Seitenklappe entlang des anderen Seitenrands und zum anderen deren in Längsrichtung einander gegenüberliegenden Enden (11) derart, daß der mit dem elastischen Teil versehene Seitenrand der zweiten Seitenklappe nach außen weist,

    e) seitliches Falzen jeweils der zweiten Seitenklappen (7), die entlang ihres einen Seitenrands mit dem elastischen Teil (8) versehen ist, so, daß der eine Seitenrand mit dem elastischen Teil nach außen gerichtet ist und

    f) Befestigen der in Längsrichtung einander gegenüberliegenden Enden (11) dieses gefalteten Abschnitts auf die Oberfläche des entsprechenden nicht gefalteten Abschnitts der zweiten Seitenklappe,

    g) Befestigen des nicht gefalteten Abschnitts (12) der zweiten Seitenklappe (7) auf der Oberfläche der zugeordneten ersten Seitenklappe (5), wobei diese zweiten Klappen (7) an den nicht gefalteten Bereichen (12) mit ihrer der ersten Seitenklappe zugewandten Fläche auf der rückseitigen Lage (1) des Hauptkörpers (6) befestigt werden.

2. Verfahren nach Anspruch 1, gekennzeichnet durch den Schritt des Wegschneidens äußerer Bereiche (15) der ersten Seitenklappen (5), so daß die jeweiligen äußeren Ränder eine gewünschte Kontur aufweisen.

3. Verfahren nach Anspruch 2, gekennzeichnet durch den weiteren Schritt des Wegschneidens äußerer Bereiche (15) der ersten und zweiten Seitenklappen (5, 7), so daß die jeweiligen Außenränder eine gewünschte Kuntur aufweisen.

4. Verfahren nach Anspruch 1, dadurch, gekennzeichnet, daß das Befestigen der zweiten Seitenklappen (7) auf die zugeordneten ersten Seitenklappen (5) entlang oder benachbart der äußeren Ränder der ersten und zweiten Seitenklappen durchgeführt wird.

5. Wegwerfunterhosen, insbesondere Windelhosen mit einem Hauptkörper (6), bestehend aus einer wasserundurchlässigen Lage (1), einer wasserdurchlässigen Lage (2) und einem zwischen diesen beiden Lagen angeordneten Saugkörper (3) und mit zwei vom Hauptkörper getrennt ausgebildeten zweiten, in Längsrichtung gefalteten Seitenklappen (7), die an ihren Außenrändern mit elastischen Teilen (8) ausgestattet sind und deren Längsenden (11) in gefaltetem Zustand miteinander verbunden und jeweils an seitlich am Hauptkörper (6) abstehenden ersten Seitenklappe (5) so festgelegt sind, daß der mit dem elastischen Teil (8) versehene Außenrand nach außen absteht, wobei

die wasserdurchlässige Oberlage (4) geringfügig größer ist als der Saugkörper (3) und eine Breite in Querrichtung aufweist, die geringer ist als die rückwärtige Lage (1), wobei die zweiten Seitenklappen (7) jeweils mit ihnen nicht-gefalteten Bereichen (12) zur rückwärtigen Lage (1) gerichtet und jeweils an der ersten Seitenklappe (5) auf der Lage (1) befestigt sind.

## Claims

1. Process for the manufacture of disposable underpants, in particular disposable nappies, comprising the steps of:

    a) formation of a main body (6) with outwardly extending first flaps (5) on opposite sides,

    b) formation of second flaps (7), separate from the main body (6),

    c) attachment of elasticated parts (8) to the second flaps (7), along one edge of each side,

    d) connection of the surface of the first flap (5) with each second flap along the other edge of the second flap and at its longitudinally oppositie ends (11), in such a way that the edge of the second flap provided with the elastic part points outwardly,

    e) sideways folding of each of the second flaps (7) which are provided along one edge with the elasticated part (8) so that the edge with the elasticated part is directed outwardly, and

    f) securing the longitudinally opposite ends (11) of this folded portion to the surface of the corresponding unfolded portion of the second flap,

    g) securing the unfolded portion (12) of the second flap (7) to the surface of the first flap (5), whereby the second flaps (7) are secured in their unfolded regions (12) with their surfaces disposed towards the first flap on the rear layer (1) of the main body (6).

2. Process as claimed in Claim 1, characterised by the step of cutting away outer regions (15) of the first flap (5), so that the outer edges have a desired form.

3. Process as claimed in Claim 2, characterised by the further step of cutting away outer regions (15) of the first and second flaps (5,7), so that the respective outer edges have a desired form.

4. Process as claimed in Claim 1, characterised in that the securing of the second flap (7) to the first flap (5) is performed along or adjacent the outer edges of the first and second flaps.

5. Disposable underpants, in particular disposable nappy, with a main body (6), comprising a water-impermeable layer (1), a water-permeable layer (2) and an absorbent body (3) disposed between these layers, and with two flaps (7), formed separately from the main body and folded longitudinally, which are provided at their outer edges with elasticated parts (8) and the longitudinal edges (11) of which are connected together in the folded condition and are secured to first flaps (5) provided at the sides of the main body (6) such that the outer edge with the elasticated part (8) protrudes outwardly, whereby

    the water-permeable overlayer (4) is slightly larger than the absorbent body (3) and has a width less than the rear layer (1), and whereby the second flaps (7) are arranged with their unfolded regions (12) disposed towards the rear layer (1) and are secured to this layer (1) of the first flaps (5).

**Revendications**

1. Procédé pour la fabrication de culottes jetables, en particulier des couches-culottes, comprenant les étapes opérationnelles suivantes :

   a) réalisation d'un corps principal (6) comportant, sur deux de ses côtés se faisant mutuellement face, des premiers rabats latéraux (5) s'étendent vers l'extérieur,

   b) réalisation de deuxièmes rabats latéraux (7), séparés du corps principal (6),

   c) fixation de parties élastiques (8) sur les deuxièmes rabats latéraux (7) te long de l'un de leurs bords latéraux ;

   d) liaison de la surface des premiers rabats latéraux (5) correspondants avec, d'une part, chacun des deuxièmes rabats latéraux le long de l'autre bord latéral de ces derniers et, d'autre part, avec leurs extrémités (11) se faisant face dans le sens longitudinal, de telle sorte que le bord latéral muni de la partie élastique du deuxième rabat latéral soit orienté vers l'extérieur,

   e) pliage latéral, respectivement, des deuxièmes rabats latéraux (7) qui sont munis de la partie élastique (8) le long de l'un de leurs bords latéraux, de telle sorte que ledit bord latéral muni de le partie élastique soit orienté Vers l'extérieur, et

   f) fixation des extrémités (11) se faisant face mutuellement en direction longitudinale de cette portion repliée sur la surface de la portion correspondante non repliée du deuxième rabat latéral,

   g) fixation de la section (12) non repliée du deuxième rabat latéral (7) sur la surface du premier rabat latéral (5) correspondant, ces deuxièmes rabats (7) étant fixés, dans les zones non repliées (12), par leur face orientée vers le premier rabat latéral, sur la couche arrière (1) du corps principal (6).

2. Procédé selon la revendication 1, caractérisé en ce qu'il consiste en une étape de découpe de zones extérieures (15) des premiers rabats latéraux (5), de telle sorte que les bords extérieurs respectifs présentent le contour souhaité.

3. Procédé selon la revendication 2, caractérisé en ce qu'il consiste en une autre étape de découpe des zones extérieures (15) des premiers et deuxièmes rabats latéraux (5, 7) de telle sorte que les bords extérieurs respectifs présentent le contour souhaité.

4. Procédé selon la revendication 1, caractérisé en ce qu'il consiste en la fixation des deuxièmes rabats latéraux (7), sur les premiers rabats latéraux (5) correspondants, le long ou au voisinage des bords extérieurs des premiers et des deuxièmes rabats latéraux.

5. Culottes jetables, en particulier couches-culottes comportant un corps principal (6) constitué d'une couche (1) imperméable à l'eau, d'une couche (2) perméable à l'eau, et d'un corps absorbant (3) disposé entre ces deux couches et comportant deux rabats latéraux (7) séparés du corps principal et repliés en direction longitudinale, lesquels sont munis, sur leurs bords extérieurs de parties élastiques (8), et dont Les extrémités Longitudinales (11), à l'état replié, sont assemblées l'une à l'autre et fixées respectivement sur des premiers rabats latéraux (5) s'écartant latéralement du corps principal (6) de telle manière que le bord extérieur muni de le partie élastique (8) s'étend vers l'extérieur, tandis que la couche supérieure (4) perméable à l'eau est légèrement plus grande que le corps absorbant (3) et présente, dons la direction transversale, une largeur qui est inférieure à celle de la couche arrière (1), chacun des deux deuxièmes rabats latéraux (7) étant orienté, par sa partie non repliée, (12) vers ta couche arrière (1) et fixé au premier rabat latéral correspondant (5), sur ladite couche arrière (1).

# FIG.1     FIG.2     FIG.3

# FIG.4

# FIG.5

# FIG.6

# FIG.7

# FIG.8

# FIG.9

# FIG.10

# FIG.11

# FIG.12

# FIG.13

# FIG.14

# FIG.15

# FIG.16

# FIG.17

# FIG.18